# EUROPEAN PATENT APPLICATION

(11) **EP 4 846 978 A2**
(43) Date of publication of application: **02.12.2026**
(21) Application number: 26212400.1
(22) Date of filing: 12.04.2017
(51) Int. Cl.: A61F 2/04

(54) **RELOAD SHAFT ASSEMBLY FOR SURGICAL STAPLER**

(30) Priority: 12.04.2016 US 201662321618 P
(62) Document number(s) of the earlier application(s) in accordance with Art. 76 EPC: 23198045.9 / 4 268 705; 21173771.3 / 3 903 688; 17719465.1 / 3 442 430
(71) Applicant: Applied Medical Resources Corporation, Rancho Santa Margarita, CA 92688 (US)
(72) Inventor: JASEMIAN, Babak D, Rancho Santa Margarita, 92688 (US); DECKER, Steven E, Rancho Santa Margarita, 92688 (US); PATEL, Atal C, Rancho Santa Margarita, 92688 (US); NELSON, Erik, Rancho Santa Margarita, 92688 (US); MCCARTHY, Andrew J, Rancho Santa Margarita, 92688 (US); ZIMMERMAN, Scott, Rancho Santa Margarita, 92688 (US); HOPKINS, Timothy M, Rancho Santa Margarita, 92688 (US); SCHOBER, Joshua M, Rancho Santa Margarita, 92688 (US); WEISS, Eric J, Rancho Santa Margarita, 92688 (US); BECERRA, Matthew M, Rancho Santa Margarita, 92688 (US); REED, Christina N, Rancho Santa Margarita, 92688 (US)
(74) Representative: Dolleymores

(57) **Abstract**

A surgical stapling system can include a reload shaft. The shaft can include an elongate tubular member with have a jaw assembly at the distal end thereof and a coupling collar at the proximal end thereof. The shaft assembly also includes an articulation joint coupling the jaw assembly to the distal end. A drive member and an articulation member extend within the tubular body of the shaft from the proximal end to the distal end. A firing member is connected to the distal end of the drive member such that advancement of the drive beam advances the firing member to close the jaw assemblies and fire staples from a reload positioned in the jaw assembly. The shaft assembly can also include a lockout mechanism to prevent a firing operation on a previously-fired reload or no reload.

## Description

### CROSS REFERENCE TO RELATED APPLICATION

This application claims the benefit of U.S. Provisional Patent Application Serial No. 62/321,618, entitled "RELOAD SHAFT ASSEMBLY FOR SURGICAL STAPLER," filed April 12, 2016, currently pending. The above-referenced application is incorporated by reference herein in its entirety.

### BACKGROUND OF THE INVENTION

### FIELD OF THE INVENTION

The present application relates generally to surgical occlusion instruments and, more particularly, to surgical staplers.

### DESCRIPTION OF THE RELATED ART

Surgical staplers are used to approximate or clamp tissue and to staple the clamped tissue together. As such, surgical staplers have mechanisms to ensure that tissue is properly positioned and captured and to drive staples through the tissue. As a result, this has produced, for example, multiple triggers and handles in conjunction with complex mechanisms to provide proper stapling of the clamped tissue. With these complex mechanisms, surgical staplers can have increased manufacturing burdens, as well as potential sources for device failure and confusion for the user. Thus, reliable stapling of clamped tissue without complex mechanisms is desired.

### SUMMARY OF THE INVENTION

In certain embodiments, a surgical stapler is provided herein. The surgical stapler comprises an elongate shaft, a jaw assembly, and a handle assembly. The elongate shaft has a proximal end and a distal end. The elongate shaft defines a longitudinal axis between the proximal end and the distal end. The jaw assembly is positioned at the distal end of the elongate shaft. The jaw assembly comprises a first jaw, a second jaw, and a plurality of staples. The jaw assembly is selectively positionable in one of a closed configuration, an open configuration, and a firing configuration. The handle assembly is positioned at the proximal end of the elongate shaft.

In certain embodiments, the elongate shaft comprises a jaw assembly at the distal end thereof coupled at an articulation joint. The articulation joint can allow articulation of the jaw assembly about an articulation range. Translation of an articulation member that extends through the elongate shaft articulates the jaw assembly. The elongate shaft further comprises a drive member extending through the elongate shaft. The drive member has a flexible segment extending through the articulation joint. A firing member is coupled to the distal end of the drive member.

In certain embodiments, the jaw assembly at the distal end of the elongate shaft comprises a reload support and an anvil pivotably coupled to the reload support. A firing member having an I-beam configuration is positioned in the jaw assembly. The jaw assembly can further comprise a lockout mechanism to prevent the firing member from being advanced unless an unfired reload is positioned in the jaw assembly.

In various embodiments, a shaft coupler can be positioned at the proximal end of the shaft. The shaft coupler can be configured to engage a coupler on a handle assembly in a bayonet connection. The bayonet connection simultaneously couples an articulation member, a drive member, and the elongate shaft. The coupler can further comprise a shaft identification mechanism. The coupler can further comprise a lock-in mechanism to retain the shaft assembly in connection with the handle assembly.

In various embodiments, a reload assembly for a surgical stapling system is provided. The reload assembly comprises an elongate shaft, a jaw assembly, a firing member, an actuation beam, and a reload lockout mechanism. The elongate shaft has a proximal end and a distal end. The elongate shaft defines a longitudinal axis extending between the proximal end and the distal end. The jaw assembly is positioned at the distal end of the elongate shaft. The jaw assembly comprises a first jaw, and a second jaw. The first jaw comprises a reload support configured to receive a staple reload. The second jaw is pivotably coupled to the first jaw. The second jaw comprises an anvil surface. The firing member is longitudinally slidable within the jaw assembly. The actuation beam is longitudinally slidable within the elongate shaft. The actuation beam has a proximal end and a distal end. The distal end of the actuation beam is coupled to the firing member. The reload lockout mechanism comprises a lockout lever pivotally coupled to the reload support and pivotable between a locked position preventing distal movement of the actuation beam relative to the elongate shaft and an unlocked position allowing distal movement of the actuation beam relative to the elongate shaft.

In various embodiments, a reload assembly for a surgical stapling system is provided. The reload assembly comprises an elongate shaft, a jaw assembly, an actuation beam, and a shaft coupler. The elongate shaft has a proximal end and a distal end and defines a longitudinal axis extending between the proximal end and the distal end. The jaw assembly is positioned at the distal end of the elongate shaft. The jaw assembly comprises a first jaw, and a second jaw. The first jaw comprises a reload support configured to receive a staple reload. The second jaw is pivotably coupled to the first jaw. The second jaw comprises an anvil surface. The actuation beam is longitudinally slidable within the elongate shaft. The actuation beam has a proximal end and a distal end. The distal end of the actuation beam is coupled to the jaw assembly. The shaft coupler is positioned at the proximal end of the elongate shaft. The shaft coupler comprises a locking member positioned therein. Thee locking member is radially outwardly advanceable by distal actuation of the proximal end of the actuation beam.

In various embodiments, a reload assembly for a surgical stapling system is provided. The reload assembly comprises an elongate shaft, a jaw assembly, an actuation beam, and a shaft coupler. The elongate shaft has a proximal end and a distal end and defines a longitudinal axis extending between the proximal end and the distal end. The jaw assembly is positioned at the distal end of the elongate shaft. The jaw assembly comprises a first jaw and a second jaw. The first jaw comprises a reload support configured to receive a staple reload. The second jaw is pivotably coupled to the first jaw. The second jaw comprises an anvil surface. The actuation beam is longitudinally slidable within the elongate shaft. The actuation beam has a proximal end and a distal end. The distal end of the actuation beam is coupled to the jaw assembly. The shaft coupler is positioned at the proximal end of the elongate shaft. The shaft coupler is configured to removably couple to a handle assembly. The shaft coupler comprises a lockout mechanism positioned therein. The lockout mechanism comprises a locking ring and a lockout member. The locking ring is rotatable about the longitudinal axis. The lockout member is radially outwardly advanceable by rotation of the locking ring.

In various embodiments, a reload assembly for a surgical stapling system is provided. The reload assembly comprises an elongate shaft, a jaw assembly, an actuation beam, an articulation link, a support link, and an articulation latching mechanism. The elongate shaft has a proximal end and a distal end and defines a longitudinal axis extending between the proximal end and the distal end. The jaw assembly is articulably coupled to the elongate shaft at the distal end of the elongate shaft. The jaw assembly comprises a first jaw and a second jaw. The first jaw comprises a reload support configured to receive a staple reload. The second jaw is pivotably coupled to the first jaw. The second jaw comprises an anvil surface. The actuation beam is longitudinally slidable within the elongate shaft to actuate the jaw assembly. The actuation beam has a proximal end and a distal end. The articulation link is longitudinally slidable within the elongate shaft to articulate the jaw assembly relative to the elongate shaft. The articulation link has a proximal end positioned adjacent the proximal end of the elongate shaft and a distal end pivotably coupled to the jaw assembly. The support link is longitudinally slidable within the elongate shaft. The support link has a proximal end extending longitudinally to a distal end pivotably coupled to the jaw assembly. The articulation latching mechanism is positioned within the elongate shaft between the proximal end and the distal end. The articulation latching mechanism has an unlatched configuration in which the articulation link and the support link are slidable within the elongate shaft and a latched configuration wherein the articulation latching mechanism engages the articulation link and the support link to prevent longitudinal sliding of the articulation link and the support link.

### BRIEF DESCRIPTION OF THE DRAWINGS

Figure 1 is a perspective view of an embodiment of surgical stapling system with the jaws in an open configuration;
Figure 2a is a perspective view of several embodiments of shaft assembly for the surgical stapling system of Figure 1;
Figure 2b is a perspective view of several embodiments of jaw assembly for the surgical stapling system of Figure 1;
Figure 3 is a perspective view of a jaw assembly at the distal end of the shaft assembly for the surgical stapling system of Figure 1;
Figure 4 is a perspective view of the jaw assembly of Figure 3 with a staple reload;
Figure 5 is a perspective view of the jaw assembly of Figure 3 with a staple reload inserted;
Figure 6A is a top view of an anvil for the jaw assembly of Figure 3;
Figure 6B is a top view of an anvil plate for the jaw assembly of Figure 3;
Figure 7 is an exploded perspective view of the anvil of the jaw assembly of Figure 3;
Figure 8 is a perspective view of the top jaw of the jaw assembly of Figure 3 in an initial state and a formed state;
Figure 9 is a perspective view of the anvil surface of the jaw assembly of Figure 3;
Figure 10 schematic diagram of staple recesses in the anvil surface of Figure 9;
Figure 11A is a perspective view of the anvil of the jaw assembly of Figure 3;
Figure 11B is a top view of the anvil of the jaw assembly of Figure 3;
Figure 12 is a perspective view of the reload support of the jaw assembly of Figure 3 with a reload partially inserted;
Figure 13 is a perspective view of the reload support of the jaw assembly of Figure 3 with a reload inserted;
Figure 14 is a side view of a closure beam of the jaw assembly of Figure 3;
Figure 15 is a partial cut-away front view of the closure beam of Figure 14 with a flange thereof positioned in a channel in the anvil of the jaw assembly of Figure 3 ;
Figure 16 is an exploded perspective view of a reload for use in the staple system of Figure 1;
Figure 17 is an upper perspective view of the reload for use in the staple system of Figure 1;
Figure 18 is a lower perspective view of the reload for use in the staple system of Figure 1;
Figure 19 is an exploded lower perspective view of the reload for use in the staple system of Figure 1;
Figure 20 is a perspective view of the reload for use in the staple system of Figure 1;
Figure 21 is a top detail view of the reload for use in the staple system of Figure 1;
Figure 22 is a perspective view of a staple pusher for the reload of Figure 16;
Figure 23 is a perspective view of a staple pusher of the reload of Figure 16;
Figure 24 is a perspective view of the reload for use in the staple system of Figure 1;
Figure 25 is a partial cut-away view of the jaw assembly of Figure 3 in a closed configuration;
Figure 26 is a perspective view of the reload of Figure 16;
Figure 27 is a perspective view of the jaw assembly of Figure 3 in a closed configuration with a reload inserted;
Figure 28 is a perspective view of the jaw assembly of Figure 3 with a reload positioned for insertion;
Figure 29 is a perspective view of the jaw assembly of Figure 3 with a reload inserted;
Figure 30 is a top view of the reload of Figure 16;
Figure 31 is a side view of the jaw assembly of Figure 3 with a reload inserted;
Figure 32A is a perspective view of a reload lockout mechanism of the shaft assembly;
Figure 32B is a side view of the reload lockout mechanism of the shaft assembly;
Figure 33 is a side view of the reload lockout mechanism of the shaft assembly in a locked configuration ;
Figure 34 is a side view of the reload lockout mechanism of the shaft assembly in an unlocked configuration;
Figure 35 is a perspective view of the distal end of the elongate shaft at an articulation joint connection with the jaw assembly of Figure 3;
Figure 36 is a partial cut-away perspective view of one embodiment of articulation joint at the distal end of the elongate shaft;
Figure 37 is a partial cut-away perspective view of one embodiment of articulation joint at the distal end of the elongate shaft;
Figure 38A is a partial cut-away top view of the articulation joint of Figure 36 in an articulated position;
Figure 38B is a partial cut-away top view of the articulation joint of Figure 36 in another articulated position;
Figure 39 is a partial cut-away perspective view of another embodiment of articulation joint at the distal end of the elongate shaft;
Figure 40 is a partial cut-away perspective view of the embodiment of articulation joint of Figure 39 at the distal end of the elongate shaft;
Figure 41 is a partial cut-away top view of the articulation joint of Figure 39 in an articulated position;
Figure 42 is a partial cut-away top view of the articulation joint of Figure 39 in another articulated position;
Figure 43 is a partial cut-away top view of the articulation joint of Figure 39 in a latched position;
Figure 44 is a side view of the proximal end of the shaft assembly positioned adjacent a handle assembly for the stapler system of Figure 1;
Figures 45A-45D are perspective views of a coupling of the proximal end of the shaft assembly to the handle assembly in a stapler system of Figure 1;
Figure 46 is an exploded perspective view of the proximal end of the shaft assembly of the stapler system of Figure 1;
Figure 47 is a cut-away side view of the proximal end of the shaft assembly positioned adjacent a handle assembly for the stapler system of Figure 1;
Figures 48A -48B are perspective views of a coupling of the proximal end of the shaft assembly to the handle assembly in a stapler system of Figure 1;
Figures 49A -49B are perspective partial cut-away views of a coupling of the proximal end of the shaft assembly to the handle assembly in a stapler system of Figure 1;
Figure 50 is a perspective partial cut-away view of the proximal end of the shaft assembly in a stapler system of Figure 1;
Figure 51 is a perspective partial cut-away view of the proximal end of the shaft assembly in a stapler system of Figure 1; and
Figure 52 is an exploded perspective view of the proximal end of the shaft assembly in a stapler system of Figure 1.

### DETAILED DESCRIPTION OF THE INVENTION

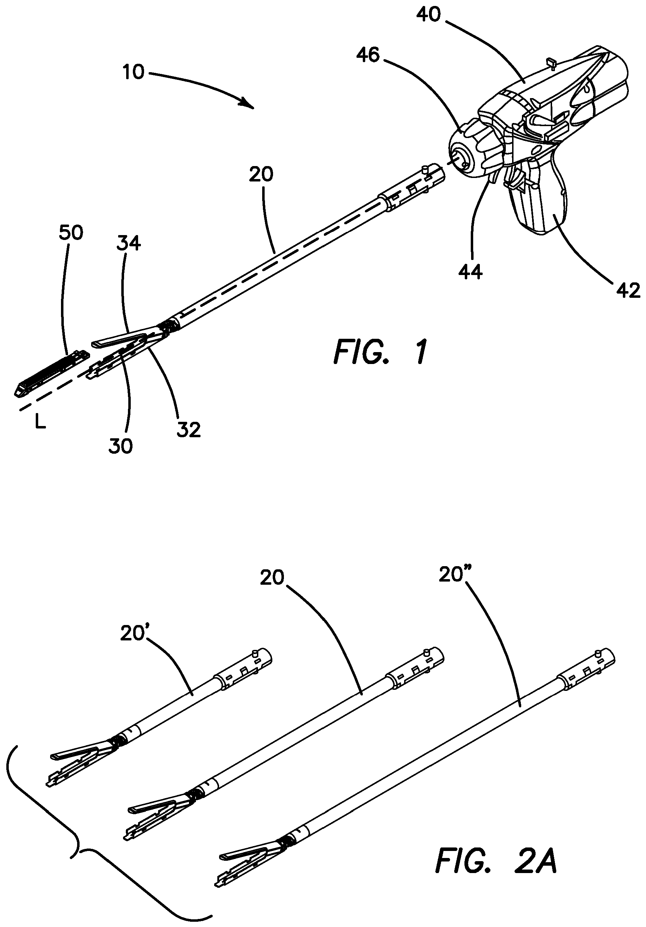

With reference to Figure 1, an embodiment of surgical stapling system is illustrated. The illustrated embodiment of surgical stapler 10 comprises an elongate shaft 20, a jaw assembly 30, and a handle assembly 40. Figure 1 illustrates the surgical stapler 10 with the jaw assembly 30 in an open configuration. A staple reload 50 can be positioned in the jaw assembly. While the illustrated surgical stapling system is illustrated with a powered handle, it is contemplated that the elongate shaft 20 and jaw assembly 30 can be interchangeably used in a stapling system including a mechanical stapler handle. For example, it is contemplated that the various embodiments of elongate shaft assembly 20 and jaw assembly 20 described herein can be used interchangeably with either the powered handle assemblies described in U.S. Patent Application Serial No. 15/486,008, entitled "SURGICAL STAPLER HAVING A POWERED HANDLE," filed April 12, 2017, currently pending, and the mechanical manually actuated handle assemblies described in U.S. Patent Application Serial No. 15/485,620, entitled "SURGICAL STAPLER HAVING ARTICULATION MECHANISM," filed April 12, 2017, currently pending. These applications are incorporated by reference herein in their entireties.

With continued reference to Figure 1, the illustrated embodiment of surgical stapler 10 can be sized and configured for use in laparoscopic surgical procedures. For example, the elongate shaft 20 and jaw assembly 30 can be sized and configured to be introduced into a surgical field through an access port or trocar cannula. In some embodiments, the elongate shaft 20 and jaw assembly 30 can be sized and configured to be inserted through a trocar cannula having a relatively small working channel diameter, such as, for example, less than 8 mm. In other embodiments, elongate shaft 20 and jaw assembly 30 can be sized and configured to be inserted through a trocar cannula having a larger working channel diameter, such as, for example, 10 mm, 11 mm, 12 mm, or 15 mm. In other embodiments, it is contemplated that certain aspects of the surgical staplers described herein can be incorporated into a surgical stapling device for use in open surgical procedures.

With continued reference to Figure 1, as illustrated, the elongate shaft 20 comprises a generally tubular member. The elongate shaft 20 extends from a proximal end to a distal end. The elongate shaft 20 defines a central longitudinal axis, L. of the surgical stapler 10 extending between the proximal end and the distal end.

With reference to Figure 2a, it is contemplated that the stapling system can include an elongate shaft having a desired length. While the features of the jaw assembly and handle coupling described herein can be substantially similar for each of these shaft assemblies, the shaft bodies can be scalable. For example, a stapling system can include a relatively short elongate shaft 20', a mid-length elongate shaft 20, or a relatively long elongate shaft 20". Each of these shaft lengths can have particular applicability for a subset of patients or procedures. For example, the short elongate shaft 20' can be useful in pediatric procedures, and the long elongate shaft 20" can be useful in bariatric procedures.

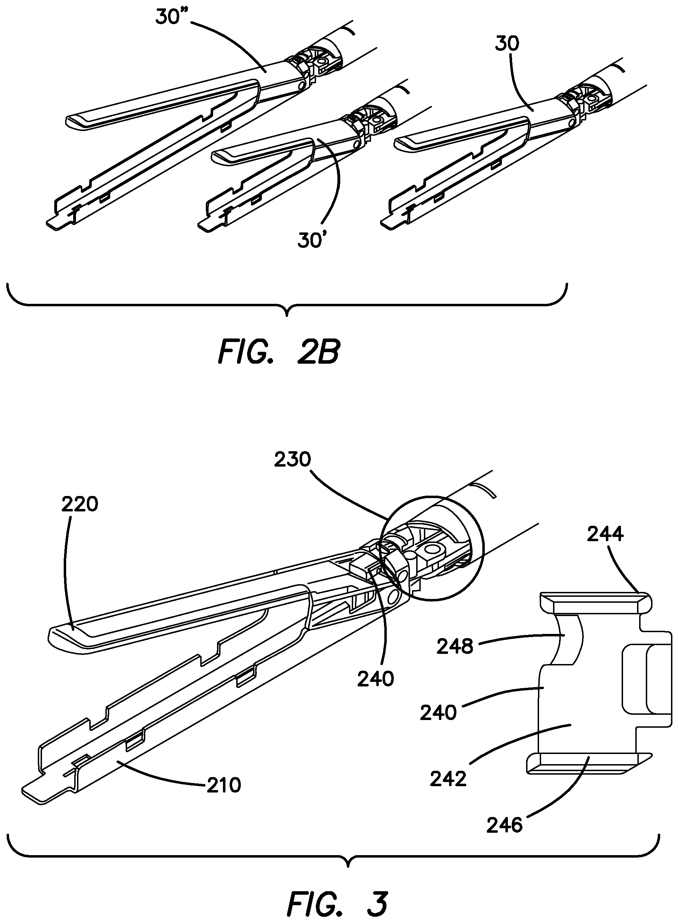

With reference to Figure 2b, it is contemplated that the stapling system can include a jaw assembly having a desired length. While the features of the jaw assembly and articulation joint described herein can be substantially similar for each of these shaft assemblies, the jaw assemblies bodies can be scalable. For example, a stapling system can include a relatively short jaw assembly 30', a mid-length jaw assembly 30, or a relatively long jaw assembly 30". Each of these jaw assemblies can have particular applicability for a subset of patients or procedures. In certain embodiments, it is contemplated that the jaw assembly have a length of approximately 45mm. In other embodiments, it is contemplated that the jaw assembly have a length of approximately 60mm.

With continued reference to Figure 1, in the illustrated embodiment, the jaw assembly 30 is coupled to the elongate shaft 20 at the distal end of the elongate shaft 20. The jaw assembly 30 comprises a first jaw 32 and a second jaw 34 pivotally coupled to the first jaw 32. In the illustrated embodiment, the jaw assembly 30 is articulable with respect to the elongate shaft 20.

With continued reference to Figure 1, in the illustrated embodiment, the jaw assembly 30 can be actuated from an open configuration (Figure 1) to a closed configuration to a stapling configuration by an actuation member or beam that is longitudinally slidable within the elongate shaft. In an initial position, the beam can be positioned at the distal end of the elongate shaft 20. With the beam in the initial position, the second jaw 34 is pivoted away from the first jaw 32 such that the jaw assembly 30 is in the open configuration. The actuation beam engages the second jaw 34 upon translation of the actuation member or beam distally along the longitudinal axis L. Translation of the actuation beam distally from the initial position a first distance can actuate the jaw assembly from the open configuration to the closed configuration. With the jaw assembly 30 in the closed configuration, the actuation beam can be returned proximally the first distance to return the jaw assembly 30 to the open configuration. A distal end of the actuation beam can advance a staple slider configured to deploy staples from the first jaw 32 such that further translation of the actuation beam distally past the first distance deploys the plurality of staples from the reload positioned in the first jaw 32.

With continued reference to Figure 1, in the illustrated embodiment, the handle assembly is coupled to the elongate shaft 20 at the proximal end of the elongate shaft 20. As illustrated, the handle assembly 40 has a pistol grip configuration with a housing defining a stationary handle 42 and a movable handle 44 or trigger pivotably coupled to the stationary handle 42. It is contemplated that in other embodiments, surgical stapler devices including aspects described herein can have handle assemblies with other configuration such as, for example, scissors-grip configurations, or in-line configurations. As further described in greater detail below, the handle assembly 40 houses an actuation mechanism configured to selectively advance an actuation shaft responsive to movement of the movable handle 44.

With reference to Figure 3, an embodiment of jaw assembly at the distal end of the shaft assembly 20 is illustrated. In the illustrated embodiment, the jaw assembly comprises a reload support 210 articulably coupled to the distal end of the shaft assembly 20 at an articulation joint 230. An anvil 220 is pivotably coupled to the reload support 210 and defines a top jaw of the jaw assembly 30. A firing member 240 can slide within the jaw assembly to initially close the anvil 220 relative to the reload support 210, then fire staples from a reload. In some embodiments, the firing member 240 has an I-beam configuration with a vertical beam 242 spanning between two horizontally-protruding flanges 244, 246. Advantageously, with an I-beam configuration, one horizontal flange 244 can engage a channel in the anvil 220 and the other flange 246 can engage a channel in the reload or reload support to close the jaw assembly then maintain a desired closed spacing of the jaw assembly when the firing member is advanced distally. In some embodiments, the firing member 240 can comprise a cutting blade 248 formed on or mounted to the vertical beam in an I-beam configuration. This cutting blade can separate tissue as staples are fired to form staple lines on both sides of the separated tissue.

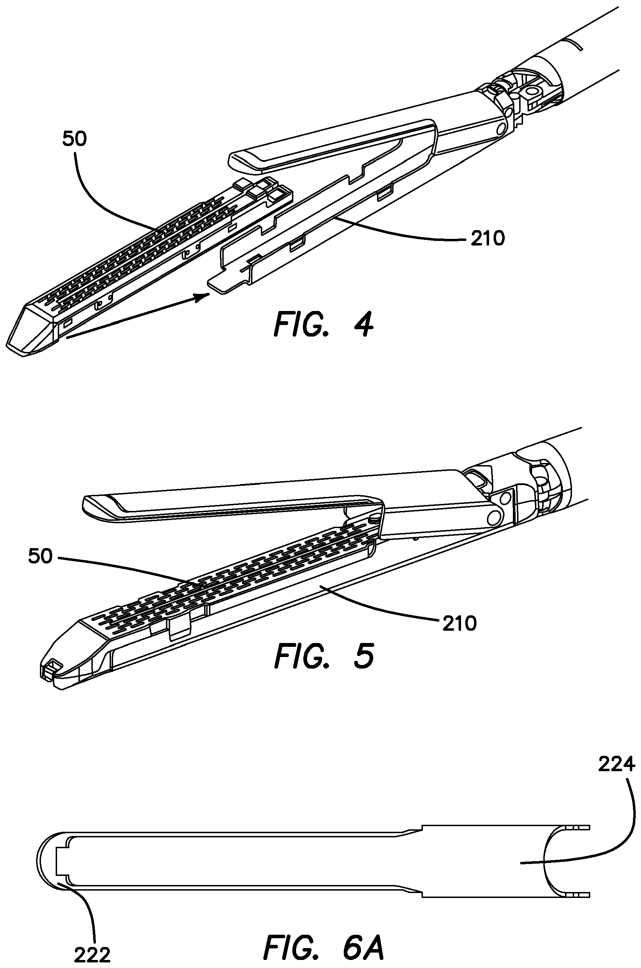

With reference to Figures 4 and 5, the reload support 210 can be sized to receive and retain a disposable reload 50. The reload 50 can be lowered and moved proximally into the reload support 210 until mating features on the reload engage corresponding features on the reload support 210.

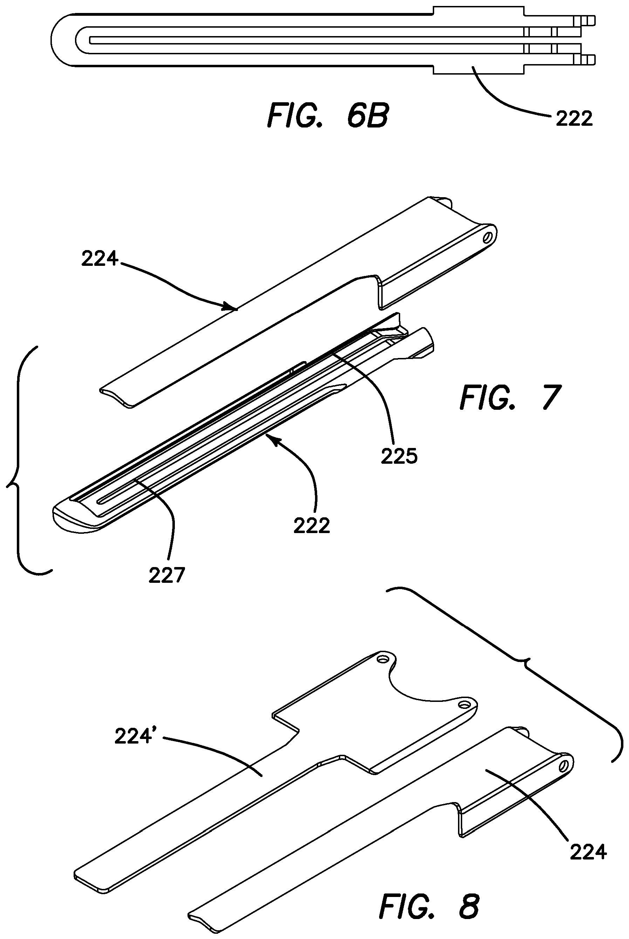

With reference to Figures 6A, 6B, 7, and 8, various aspects of the anvil 220 of the jaw assembly 30 are illustrated. In certain embodiments, the anvil 220 comprises an anvil plate 222 coupled to a top surface 224. The anvil plate can comprise a longitudinal channel 225 formed therein in which a horizontal flange of the firing member rides and a longitudinal slot 227 formed through the longitudinal channel 225 in which the vertical beam of the firing member rides. The top surface 224 can be formed of a sheet of material that is subsequently formed to overly the anvil plate. (Figure 8 illustrates the flat sheet 224' and shaped top surface 224). Advantageously, the addition of the top surface 224 to the anvil plate 222 enhances the strength of the anvil of the jaw assembly.

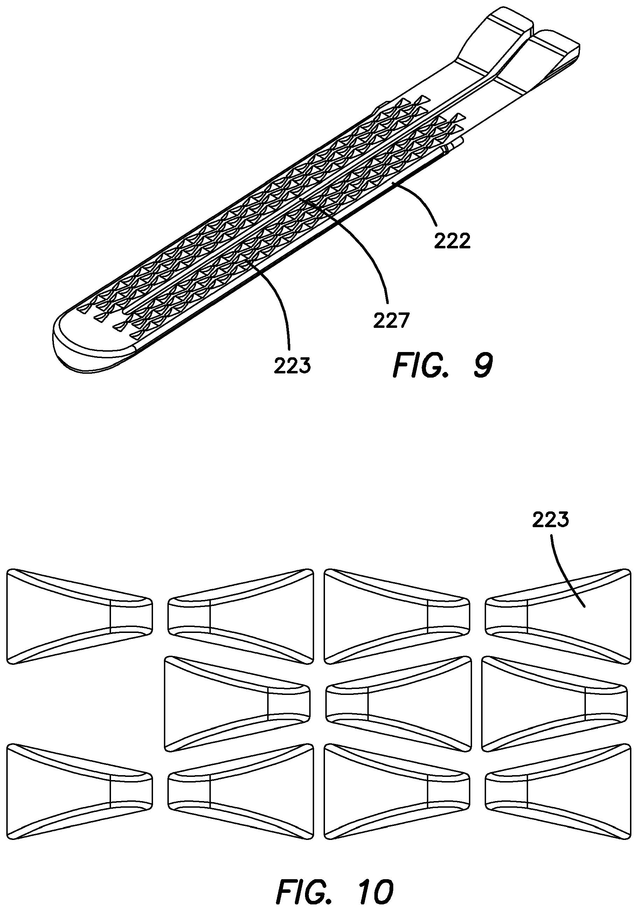

With reference to Figures 9 and 10, various aspects of the anvil plate 222 of the jaw assembly 30 are illustrated. The anvil plate comprises a plurality of staple forming pockets 223 thereon. In the illustrated embodiment, the staple forming pockets 223 are positioned in two arrays of three rows with the arrays positioned on either side of the slot for the firing member. Thus, the stapler can form two sets of three linear rows of staples with the sets separated by divided tissue. In other embodiments, it is contemplated that the anvil can include staple forming pockets configured to form other numbers and configurations of staples. The staple forming pockets have a tapered configuration with a relatively large staple entry side narrowing to a relatively small staple formation side. Advantageously, this tapered configuration can guide staples to complete formation and reduce the incidence of poorly formed staples. Adjacent rows of staples can be longitudinally offset from one another such that the relatively wide entry sides of all of the rows are offset from one another to reduce the overall width of the sets of staple rows.

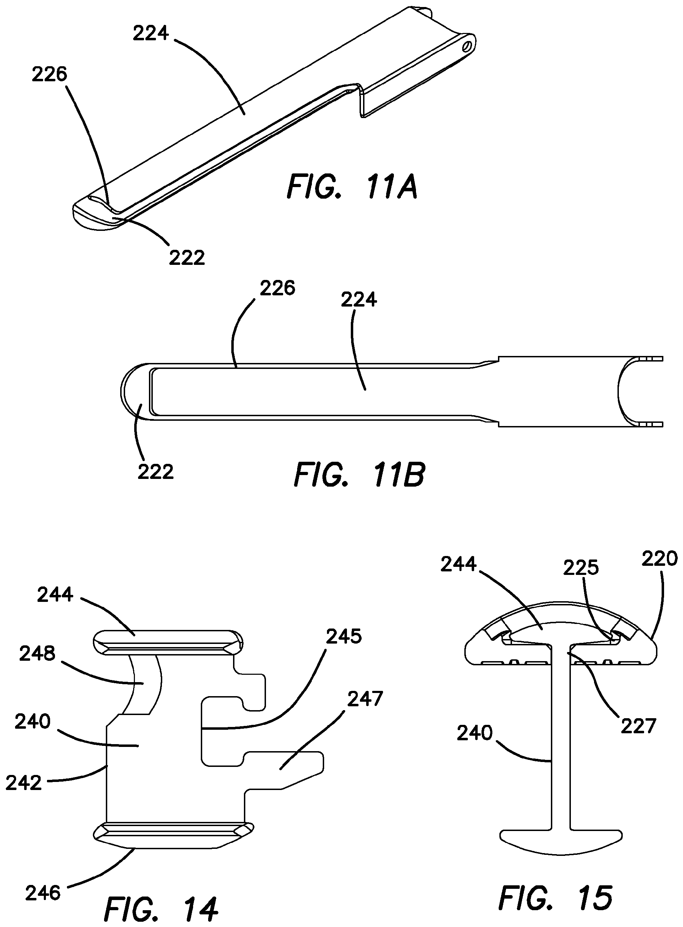

With reference to Figures 11a and 11b, in certain embodiments of anvil 220, the top surface 224 can be coupled to the anvil plate 222 by a welding operation along a weld line 226. Advantageously, this closed anvil formed by the welding operation covers the channel for the firing member.

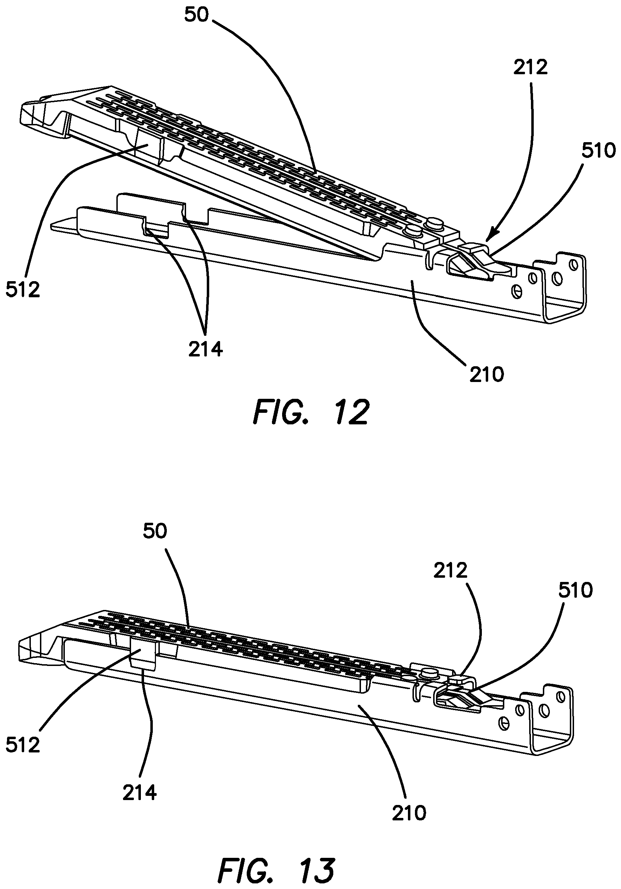

With reference to Figures 12 and 13, insertion of a reload 50 in the reload support 210 is illustrated. The reload support can comprise proximal jaw tabs 212 that protrude radially inwardly from side walls of the reload support 210 adjacent the proximal end thereof. The reload can comprise a relatively short, tapered proximal deck 510 sized to be positioned under and retained by the proximal jaw tabs. Moreover, the reload 50 can include retention tabs 512 protruding laterally outwardly adjacent a distal end thereof. The reload support 210 can comprise a corresponding pair of retention recesses 214 sized and configured to receive the retention tabs when the reload is positioned in the reload support.

With reference to Figures 14 and 15, an embodiment of firing member 240 having an I-beam configuration is illustrated. In the illustrated embodiment, The firing member comprises a vertical beam 242 having a cutting blade formed therein at a leading edge. The cutting blade comprises a curved cutting blade 248. A trailing edge of the firing member 240 comprises a drive member interface 245 such as a cutout or protrusion to allow the firing member to be securely coupled with the drive member extending through the elongate shaft. The trailing edge of the firing member 240 can further comprise a lockout interface 247, such as a proximally extending 'tail' that can position a reload lockout in an unlocked configuration when the firing member is in a proximal position. The firing member further comprises an upper horizontal flange 244 configured to ride in the channel 225 of the anvil and a lower horizontal flange 246 configured to engage the reload or reload support. As illustrated in Figure 15, although the firing member has a general I-beam configuration, in some embodiments the horizontal flanges are curved or tapered such to conform with a shape of the channel 225 in the anvil. In some embodiments, the firing member 240 can further be configured to reduce friction during a firing sequence such as by surface finishing operations, addition of a film lubricant, or deposition of a low-friction surface on the firing member, channel, or both.

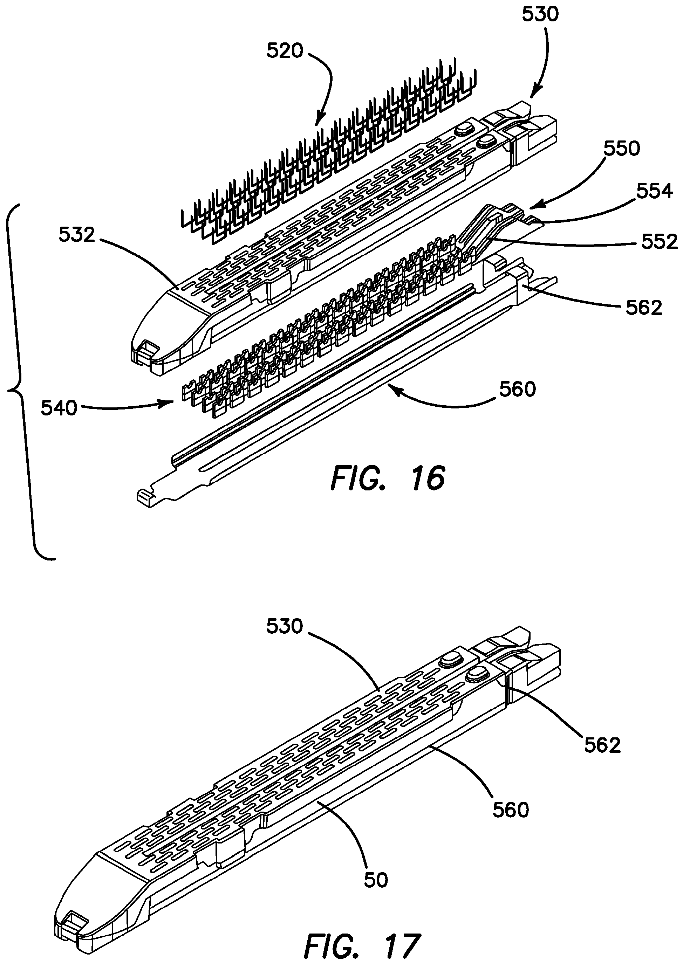

With reference to Figures 16-19, an embodiment of reload 50 for use in the stapling system is illustrated. The reload 50 comprises a plurality of staples 520 positioned in a corresponding plurality of staple pockets 532 formed in a cartridge 530. The staple pockets 532 are arranged in two sets of three rows each with each set separated by a slot formed through the cartridge 530. The staples 520 rest in a plurality of staple pushers 540 underlying the staple pockets 532. A slider 550 having a ramp 552 corresponding to each row of staple pusher 540 and a lockout tail 554 is positioned at the proximal end of the reload. The slider 550 is longitudinally slidable within the reload responsive to movement of the firing member. A jacket 560 underlies the cartridge and maintains the staples and staple pushers in the staple pockets. The jacket can have protruding hooks 562 to engage the cartridge.

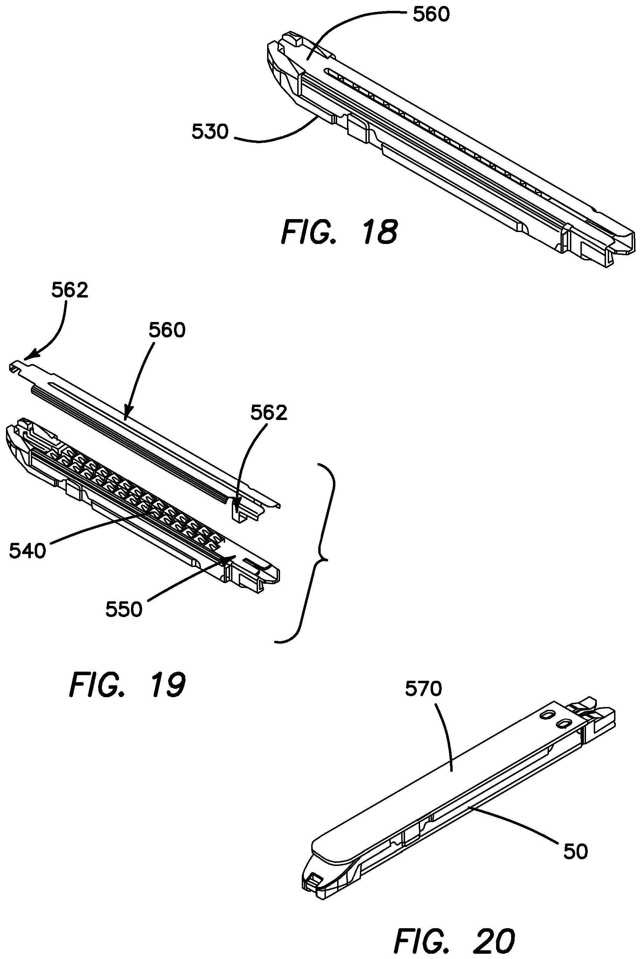

With reference to Figure 20, in some embodiments, the reload 50 can include a shipping cover 570 covering an upper surface of the cartridge. Advantageously, the shipping cover 570 can prevent one or more of the staples from becoming dislodged from or misaligned within the staple pockets before the reload is used. The shipping cover 570 is removed before the reload 50 is positioned in the reload support.

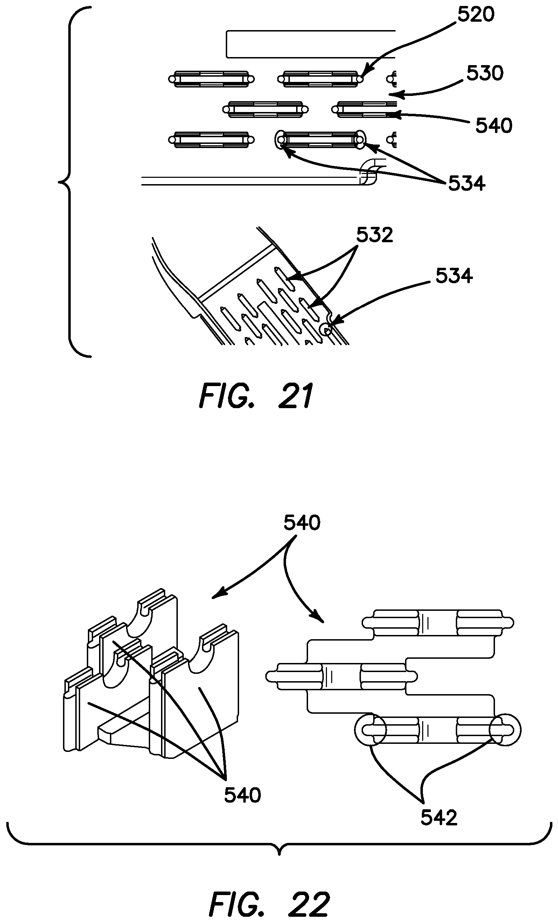

With reference to Figures 21-23, in some embodiments, the reload 50 can include certain staple alignment and retention features. For example the staple pockets 532 formed in the cartridge 530 can include staple guides 534 at ends thereof to receive legs of the staples 520 positioned therein. The staple pushers 540 can additionally include nubs 542 sized and configured to ride in the staple guides 534. As illustrated, in certain embodiments, the staple pushers 540 can be formed in groups of three such that one staple pusher 540 can push a single staple in each of three adjacent rows of staples. Moreover, an upper surface 544 of each of the staple pushers 540 can include a staple saddle configuration to relatively securely receive a staple. Secure positioning of the staples 520 in the staple pushers 540 and engagement of the staple legs and nubs 542 of the staple pushers with the staple guides can advantageously reduce the incidence of misaligned or malformed staples.

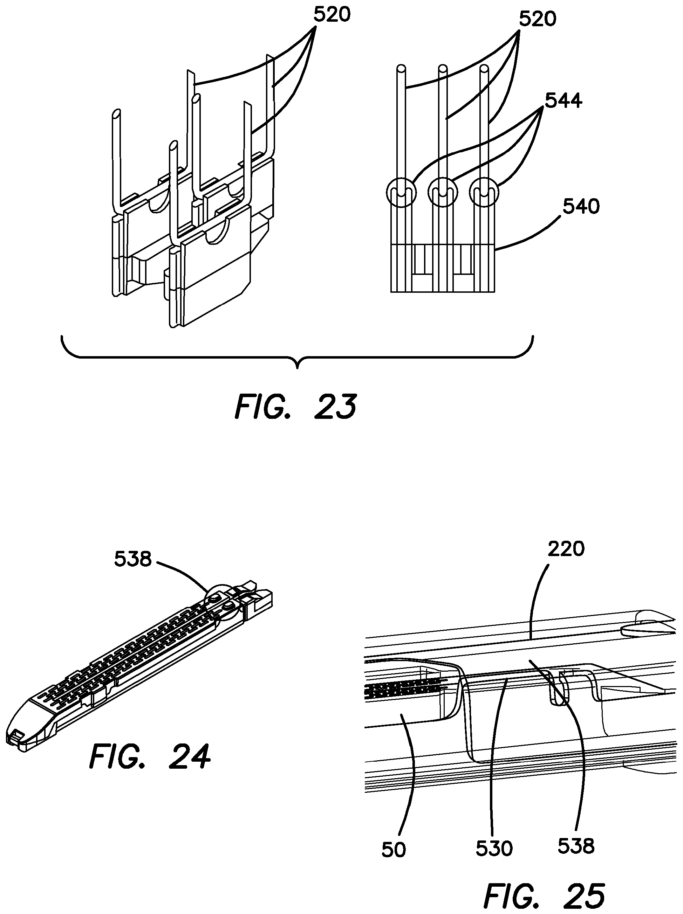

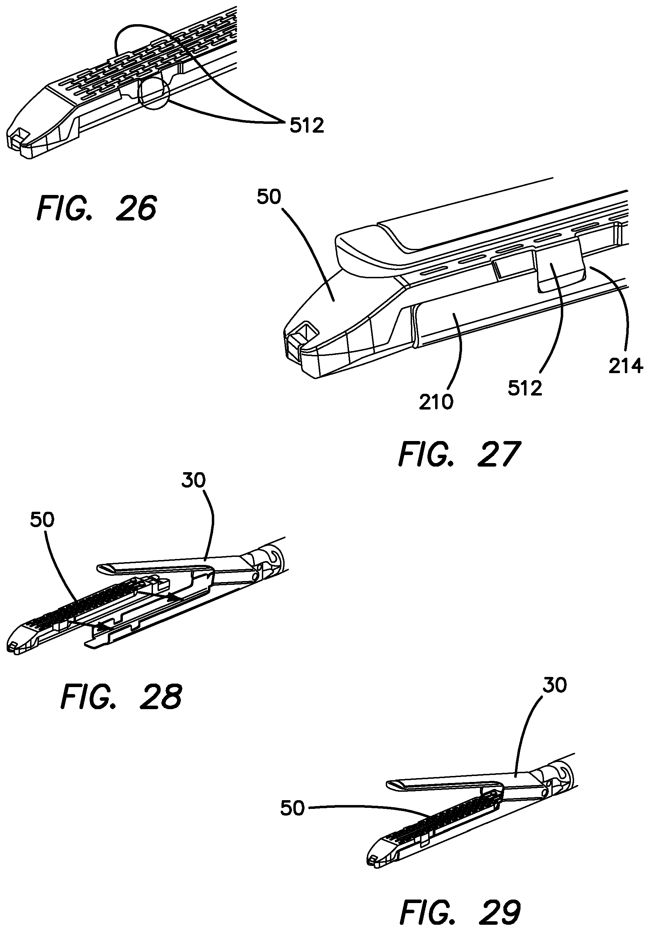

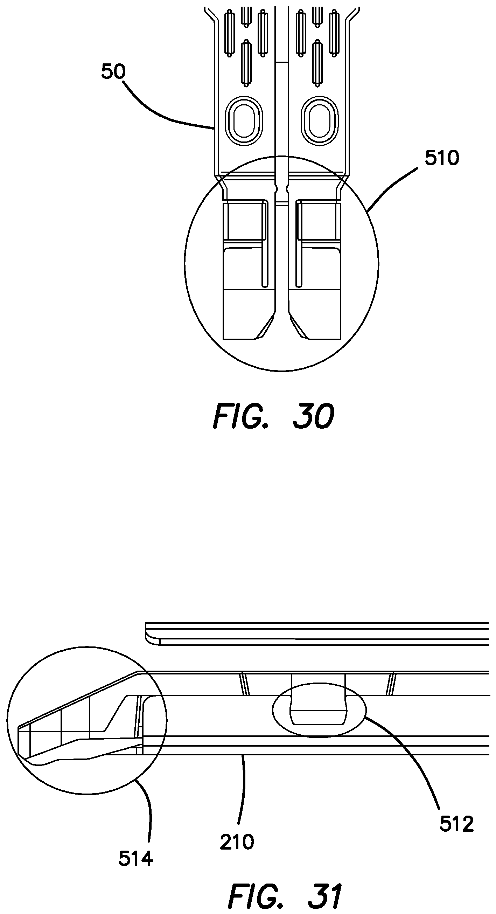

With reference to Figures 24-31, in various embodiments, the reload 50 and jaw assembly can be configured to be securely coupled to one another to align the staple pockets on the reload 50 with the staple forming pockets on the anvil and maintain the position of the reload 50 in the jaw assembly during staple firing. The reload 50 can include upwardly protruding bosses 538 at a proximal end thereof (Figure 24) that define a tissue gap between the anvil 220 and an upper surface of the cartridge 530 of the reload 50 with the jaw assembly in a closed configuration (Figure 25). Moreover, the retention tabs 512 formed adjacent the distal end of the reload (Figure 26) are positioned within recesses 214 of the reload support 210 and prevent the reload from shifting distally during a firing operation. Thus, the reload 50 can be rapidly and securely coupled to the reload support 210 (Figures 28-29). Additionally, a proximal end of the cartridge 530 can taper to a reduced height to further facilitate placement on the reload support (Figure 30). Furthermore, the cartridge can be configured with a lowered distal end 514 having a profile protruding below the reload support (Figure 31). This lowered profile ensures secure engagement of the reload with the reload support.

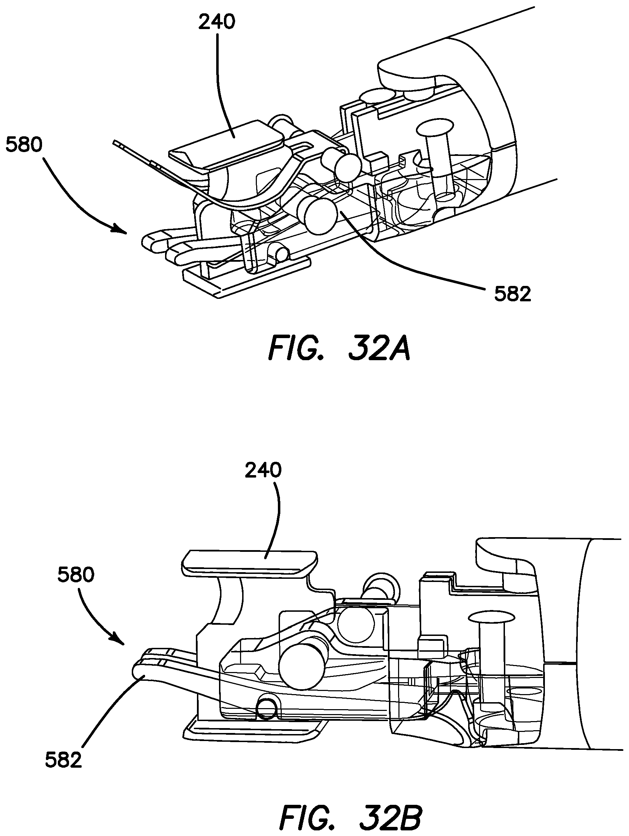

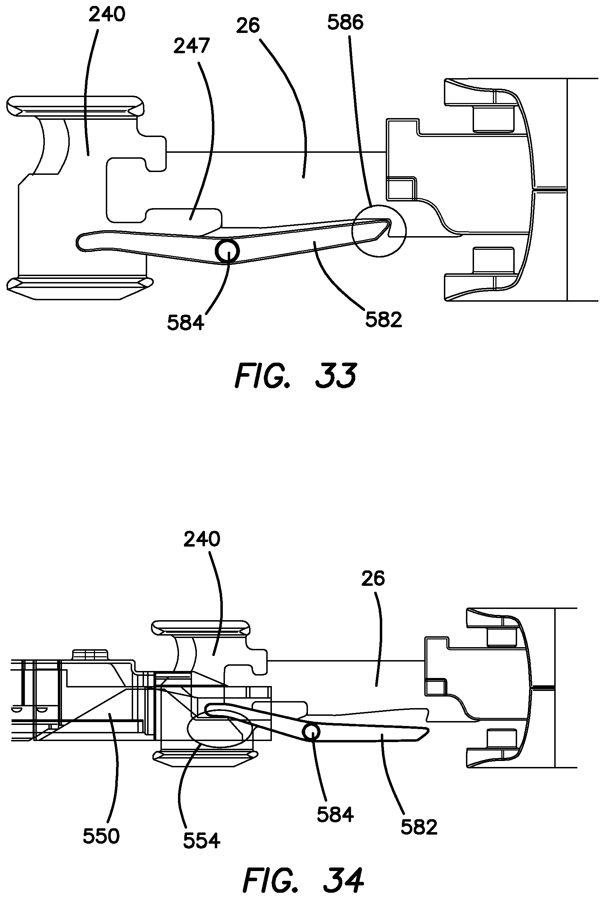

With reference to Figures 32A, 32B, and 33-34, in certain embodiments, the jaw assembly can comprise a reload lockout mechanism 580. The reload lockout mechanism 580 can prevent advancement of the firing member if no reload is positioned within the jaw assembly or if an empty reload is positioned within the jaw assembly. The reload lockout mechanism 580 includes a lockout lever 582 pivotally coupled to the reload support. An axis defined by the pivot extends generally transverse to the longitudinal axis of the elongate shaft. With the firing member 240 fully retracted such that the jaw assembly is in an open configuration, a tail 247 extending proximally from the firing member 240 maintains the lockout lever 582 pivoted to the unlocked position. In the illustrated embodiment, a proximal portion of the lockout lever 582 proximal the pivot is forked or bifurcated to receive the firing member 240 therein such that the tail 247 can act on a surface of the lockout lever 582 distal the pivot. If no reload is inserted, an attempt to advance the firing member 240 will allow the lockout lever to pivot about a pivot point 584 from the unlocked position to the locked position as the tail 247 of the firing member is advanced distally along the lockout lever. (Figure 33). With the lockout lever 582 in the locked position, a proximal, locking end 586 of the lockout lever interferes with a lock recess on the drive member 26, preventing further distal movement of the drive member.

With continued reference to Figures 32A, 32B, and 33-34, if an unfired reload is inserted into the reload support (Figure 34), a tail 554 extending proximally from the slider 550 engages a distal end of the lockout lever 582. As illustrated, the tail 554 acts on a lower surface of a distal portion of the lockout lever 582 distal the pivot point. This engagement of the slider tail 554 with the distal end of the lockout lever 582 pivots the proximal end of the lookout lever 582 away from the drive member 26 even once the tail 247 of the firing member 240 is no longer acting on the proximal portion of the lockout lever. Accordingly, the drive member 26 and firing member 240 can be distally advanced to fire the staples from the reload. Upon completion of a firing stroke, the slider 550 remains at a distal end of the reload. Thus if the jaw assembly is returned to the open configuration, withdrawing the firing member, the fired reload should be removed and a new unfired reload should be inserted to unlock the reload lockout.

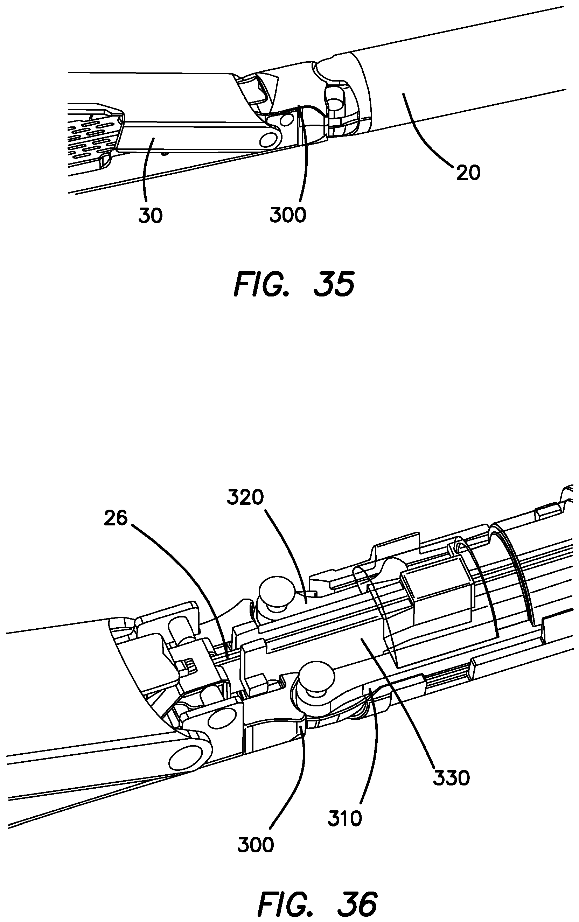

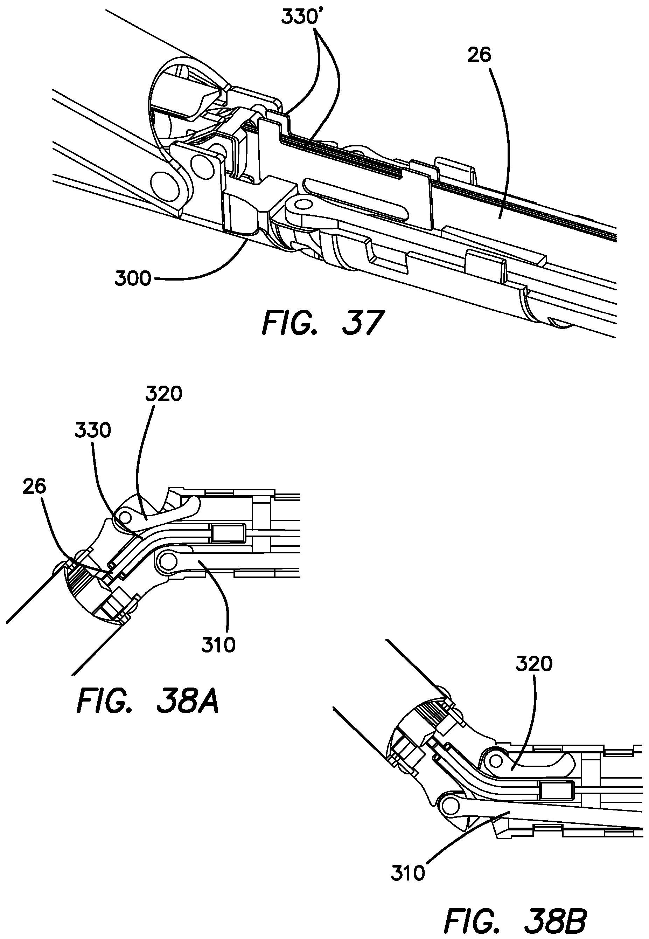

With reference to Figures 35-37, an embodiment of articulation joint 300 to couple the jaw assembly 30 to the distal end of the elongate shaft 20 is illustrated. In the illustrated embodiment, the articulation joint 300 comprises an articulation rod 310 pivotably coupled to the jaw assembly laterally offset from a central longitudinal axis of the shaft assembly. A pivot joint is positioned along the central longitudinal axis. The articulation joint 300 further comprises a support link 320 pivotably coupled to the jaw assembly laterally offset from the central longitudinal axis of the shaft and opposite the articulation rod. The drive beam 26 extends longitudinally along the central longitudinal axis between the articulation rod 310 and the support link 320. At least a segment of the drive beam 26 extending through the articulation joint 300 is flexible. In some embodiments, the drive beam 26 can be coupled to a flexible segment comprising a stack of shim material, which is flexible while maintaining desired force transmission capabilities for a staple firing operation. The articulation joint can further comprise one or more drive member bearings 330 positioned laterally outwardly of the drive beam 26. In some embodiments, the drive bearings 330 can comprise a flexible plastic material (Figure 36). In other embodiments, the drive bearings 330' can be comprised of a metal shim material (Figure 37). Advantageously, the metal shim drive bearing 330' can be keyed into the shaft to provide support to the flexible segment of the drive member. Moreover, the metal shim bearings can have a relatively low profile configuration. The metal shim bearings can include a low friction coating such as a TEFLON coating to reduce friction during a firing.

With reference to Figures 38A-38B, articulation of the articulation joint to position the jaw assembly in a first articulation position and a second articulation position are illustrated. The articulation rod 310 can be translated proximally (Figure 38A) or distally (Figure 38B) relative to the shaft. The lateral offset positioning of the articulation rod 310 articulates the jaw assembly relative to the shaft responsive to translation of the articulation rod. The support link 320 opposite the articulation rod 310 is passive, but can guide articulation motion of the jaw assembly and can advantageously assist in maintaining the flexible portion of the drive beam 26 towards the center of the shaft at the articulation joint, preventing the flexible portion of the drive beam 26 from buckling at the articulated bend at the articulation joint. In other embodiments, the articulation joint can include two articulation rods instead of an articulation rod and support link. In embodiments with two articulation rods, an articulation latch mechanism can be positioned in the shaft to prevent undesired articulation once a staple firing operation has commenced. For example, a latch or brake mechanism can retain the articulation rods from further movement once the drive beam 26 is translated distally.

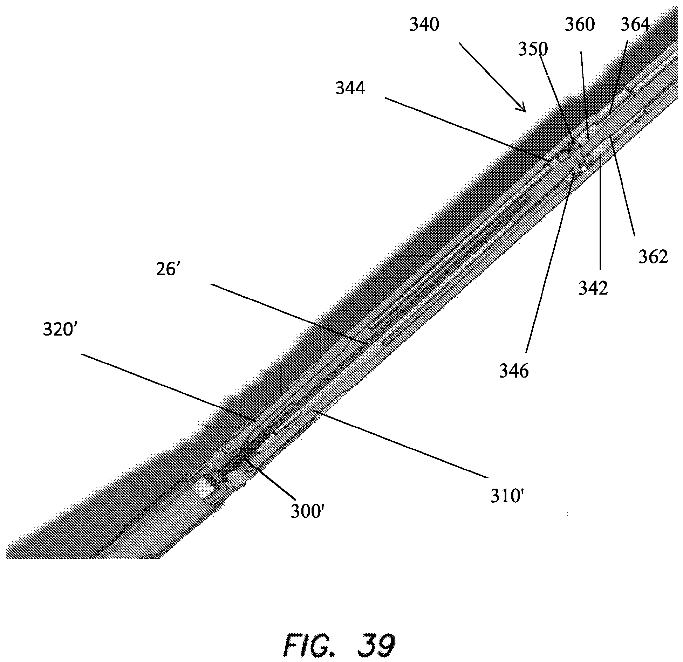

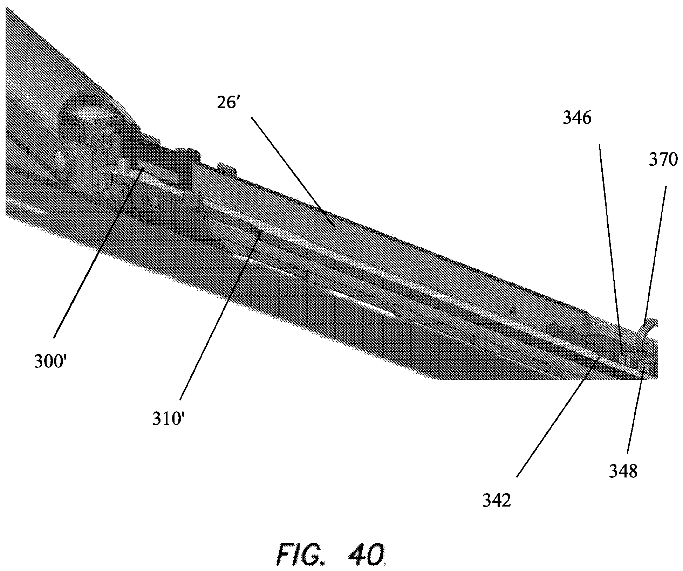

With reference to Figures 39-40, another embodiment of articulation joint 300' to couple the jaw assembly 30 to the distal end of the elongate shaft 20 is illustrated. The articulation joint 300' comprises an articulation latch mechanism 340 positioned in the elongate shaft. In the illustrated embodiment, the articulation joint 300' comprises an articulation rod 310' pivotably coupled to the jaw assembly laterally offset from a central longitudinal axis of the shaft assembly. A pivot joint is positioned along the central longitudinal axis. The articulation joint 300' further comprises a support link 320' pivotably coupled to the jaw assembly laterally offset from the central longitudinal axis of the shaft and opposite the articulation rod. The drive beam 26' extends longitudinally along the central longitudinal axis between the articulation rod 310' and the support link 320'. At least a segment of the drive beam 26' extending through the articulation joint 300' is flexible. In some embodiments, the drive beam 26' can be coupled to a flexible segment comprising a stack of shim material, which is flexible while maintaining desired force transmission capabilities for a staple firing operation. The articulation joint can further comprise one or more drive member bearings 330 positioned laterally outwardly of the drive beam 26'. In some embodiments, the drive bearings 330 can comprise a flexible plastic material (Figure 36). In other embodiments, the drive bearings 330' can be comprised of a metal shim material (Figure 37). Advantageously, the metal shim drive bearing 330' can be keyed into the shaft to provide support to the flexible segment of the drive member. Moreover, the metal shim bearings can have a relatively low profile configuration. The metal shim bearings can include a low friction coating such as a TEFLON coating to reduce friction during a firing.

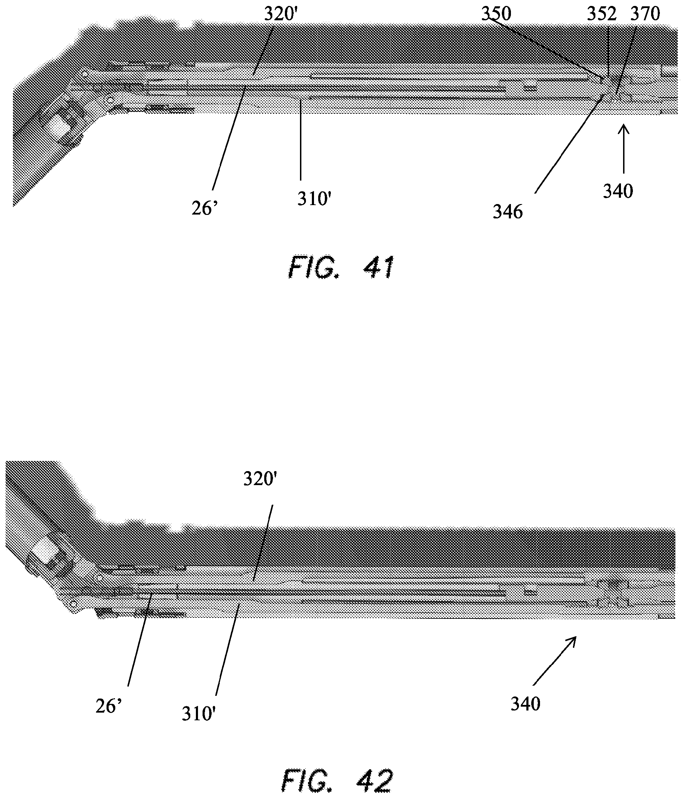

With reference to Figures 41-42, articulation of the articulation joint to position the jaw assembly in a first articulation position and a second articulation position are illustrated. The articulation rod 310' can be translated proximally (Figure 41) or distally (Figure 42) relative to the shaft. The lateral offset positioning of the articulation rod 310' articulates the jaw assembly relative to the shaft responsive to translation of the articulation rod. The support link 320' opposite the articulation rod 310' is passive, but can guide articulation motion of the jaw assembly and can advantageously assist in maintaining the flexible portion of the drive beam 26' towards the center of the shaft at the articulation joint, preventing the flexible portion of the drive beam 26' from buckling at the articulated bend at the articulation joint. In other embodiments, the articulation joint can include two articulation rods instead of an articulation rod and support link.

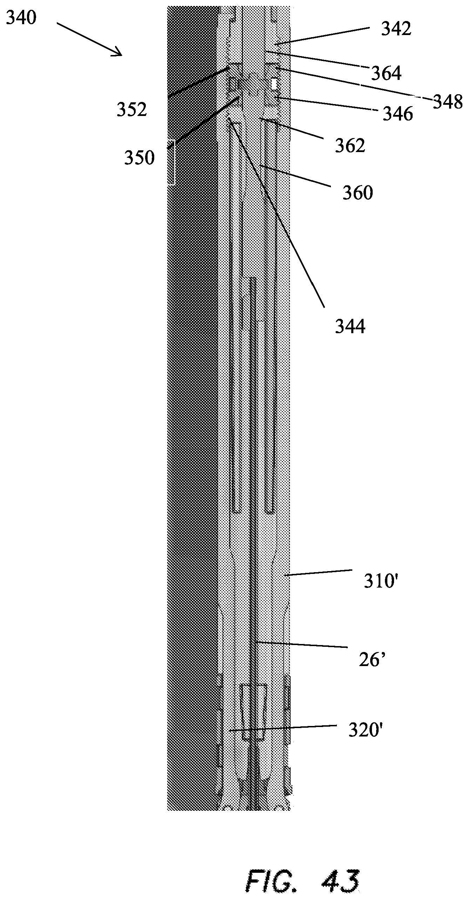

With reference to Figures 39-43, the articulation latch mechanism 340 or brake mechanism of the articulation joint 300' can retain the articulation rod and support link from further movement once the drive beam 26' is translated distally. In the illustrated embodiment, the latch mechanism 340 is positioned within the elongate shaft between the proximal end and the distal end thereof. The articulation latching mechanism 340 has an unlatched configuration in which the articulation rod and the support link are slidable within the elongate shaft. Thus, with the articulation latching mechanism in the unlatched configuration, a user can articulate the jaw assembly relative to the elongate shaft by operation of an articulation control on the handle assembly. The articulation latching mechanism 340 further comprises a latched configuration (Figure 43), wherein the articulation latching mechanism engages the articulation rod and the support link to prevent longitudinal sliding of the articulation link and the support link relative to the elongate shaft. Thus, in the latched configuration, the jaw assembly is retained in an articulated position and the user is prevented from articulating the jaw assembly relative to the elongate shaft.

With continued reference to Figures 39-43, in the illustrated embodiment, the articulation latching mechanism 340 comprises a first latch surface, such as a first plurality of teeth 342 formed on the articulation rod 310'. As illustrated, the first plurality of teeth 342 is positioned within the elongate shaft between the proximal end and the distal end of the articulation rod 310'. The articulation latching mechanism 340 can further comprise a second latch surface, such as a second plurality of teeth 344 formed on the support link 320'. As illustrated, in the embodiment of elongate shaft assembly having a latching articulation mechanism, the support link 320' can extend proximally within the shaft through the articulation latching mechanism 340. In the illustrated embodiment, the second plurality of teeth 344 is positioned between the proximal end of the support link and the distal end of the support link adjacent the proximal end of the support link 320'.

In the illustrated embodiment, the articulation latching mechanism 340 further comprises a first shoe 346 having a mating surface such as a first pawl surface 348 formed thereon. The first pawl surface 348 is sized and configured to be engageable with the first plurality of teeth 342. The first shoe 346 can have a deployment surface opposite the mating surface, the deployment surface is in sliding engagement with the drive beam 26'. The articulation mechanism 340 can further comprise a second shoe 350 having a mating surface such as a second pawl surface 352 formed thereon. The second pawl surface 352 is sized and configured to be engageable with the second plurality of teeth 344. The second shoe 350 can have a deployment surface opposite the mating surface, the deployment surface in sliding engagement with the drive beam 26'. The articulation latching mechanism 340 can further comprise a latching profile formed on the drive beam 26' between the proximal end and the distal end thereof and positioned within the elongate shaft. In the illustrated embodiment, the drive beam 26' comprises a recess segment 360 formed therein, a tapered or ramped segment 362 proximal the recess segment, and a latching segment 364 proximal the ramped segment. The recess segment 360 has a first width in a direction generally perpendicular to the longitudinal axis of the elongate shaft, and the latching segment 364 has a second width greater than the first width. The articulation latching mechanism can further comprise a biasing member such as a spring clip 370 coupled to the first and second shoes and biasing the shoes 346, 350 out of engagement with the first and second pluralities of teeth 342, 344. The spring clip can also maintain engagement of the deployment surfaces of the shoes 346, 350 with the latching profile of the drive beam 26'.

With continued reference to Figures 39-43, in operation, the articulation latching mechanism 340 can initially be positioned in the unlatched configuration (Figures 39-42) such that the jaw assembly can be articulated to a desired orientation relative to the elongate shaft. In this initial positioning, the drive beam 26' is in a proximal position relative to the elongate shaft, corresponding to an open or partially closed configuration of the jaw assembly. In the unlatched configuration, the first and second shoes 346, 350 are positioned adjacent the recess segment 360 of the drive beam 26' in a radially inward position. Once a desired articulated position of the jaw assembly has been selected, a user can proceed to close and fire the jaw assembly, resulting in distal actuation of the drive beam 26' relative to the elongate shaft. This distal movement of the drive beam 26' advances the ramped and latching segments 362, 364 over the deployment surfaces of the first and second shoes 346, 350, advancing the shoes radially outwardly. (Figure 43). With the first and second shoes 346, 350 in the radially outward configuration, the first pawl surface 348 engages the first plurality of teeth 342, and the second pawl surface 348 engages the second plurality of teeth 342 to configure the articulation latch mechanism in the latched configuration. Opening the jaw assembly after a firing sequence will reverse the sequence and return the articulation latch to the unlatched configuration. Thus, desirably, actuation of the drive member 26' to close and fire the jaw assembly automatically latches an articulated position of the jaw assembly. Advantageously, this latching can reduce or prevent any tendency of the jaw to 'wag' relative to the elongate shaft as the drive beam is advanced around and retracted through the articulation bend. While the illustrated embodiment of actuation latching mechanism includes meshing arrays of teeth on the shoes and actuation rod and support link that define a plurality of discreet latched positions, it is contemplated that in other embodiments, the shoes, actuation rod, and support link can be configured to frictionally engage to define a continuous array of latched articulation positions. Moreover, while the illustrated embodiment includes two shoes each engageable with a corresponding plurality of teeth, in other embodiments, a single shoe can be advanceable to engage a single plurality of teeth on the articulation rod or support link.

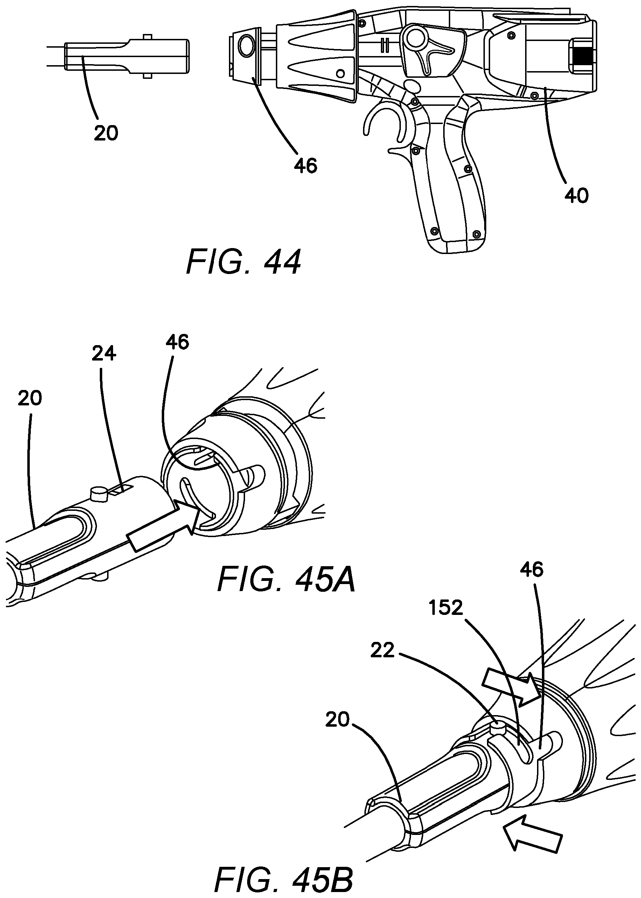

With reference to Figures 44 and 45A-45D, a coupler 46 at the distal end of the handle assembly 40 can be coupled to the proximal end of the shaft assembly 20. The coupler 46 can include a bayonet connection with a lock-in. In the illustrated embodiment, the reload shaft 20 to handle 40 connection comprises a bayonet style connection, in which a user axially aligns and inserts the reload shaft 20 into the handle 40 and rotates the reload shaft 20 approximately 90 degrees to connect. This bayonet connection operatively couples two mechanical functions of the reload shaft 20 to corresponding actuators of the handle 40. When the bayonet connection is fully coupled, an articulation member within the shaft 20 is coupled to an articulation adapter of the handle and a drive member within the shaft 20 is coupled to the actuation adapter. Furthermore, the handle 40 and shaft 20 can be configured with a latch mechanism at the coupler 46 to prevent a user from removing the shaft 20 once the actuation adapter and drive member has been activated. Moreover, the connection at the coupler 46 can include a reload identifying mechanism such that the control system of the handle can detect if a reload shaft is connected, and if so what the attached jaw length of the reload is. It is contemplated that the handle can be used with reload shafts 20 including different length jaw assemblies. In some embodiments the same handle 40 can be used with either 45 mm or 60mm length jaw assemblies.

In Figure 45A, the shaft 20 is positioned in alignment with the coupler 46 on the handle, and a release knob of the coupler 46 is withdrawn to expose a bayonet channel 152 of the coupler 46 on a rotation insert of the coupler 46. The shaft 20 can include a retention post 22 or boss positionable within the bayonet channel 152. In the illustrated embodiment, the shaft includes two bosses positioned 180 degrees apart on the outer surface thereof and the coupler 46 includes a corresponding two bayonet channels 152. It is contemplated that in other embodiments, other numbers and configurations of bosses and bayonet channels can be used to provide a desired connection strength and ease of alignment.

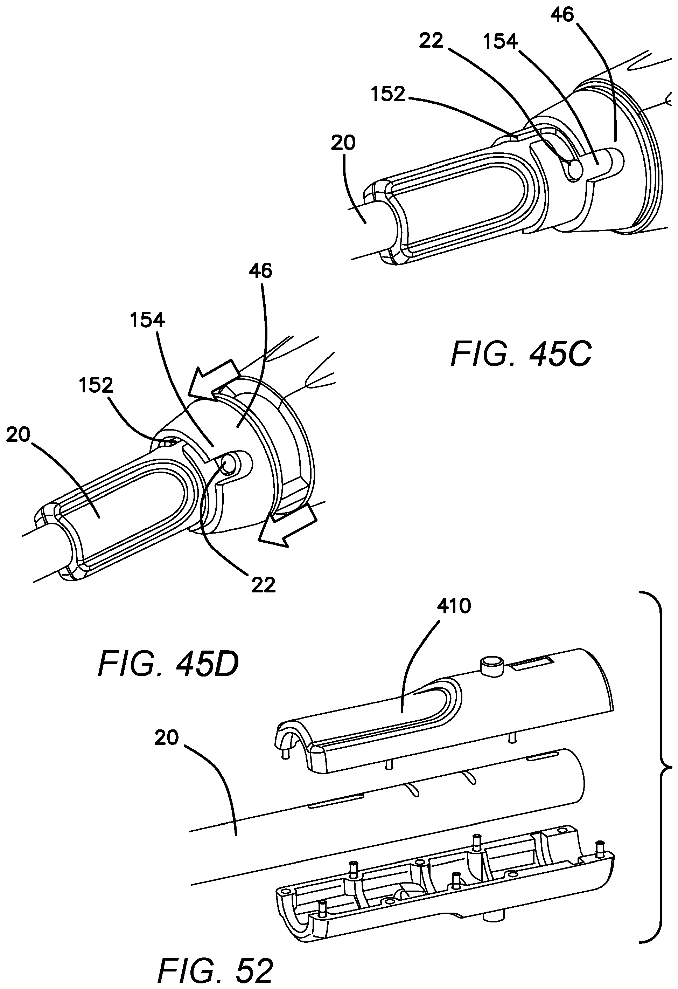

With reference to Figure 45B, the retention post 22 of the shaft is positioned within the bayonet channel 152. With reference to Figure 45C, the reload shaft 20 has been rotated 90 degrees relative to the handle such that the retention post 22 of the shaft has reached a connected end of the bayonet channel 152. With reference to Figure 45D, the release knob of the coupler is released to allow a retention recess 154 on the release knob to retain the retention post 22 of the reload shaft 20.

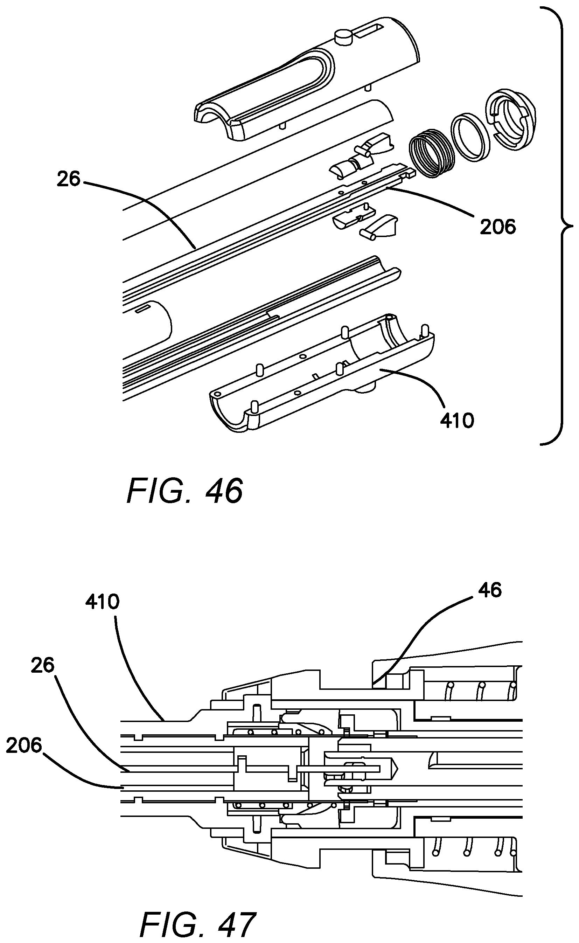

With reference to Figures 46 and 47, the shaft assembly can include a tubular shaft with the drive member or drive beam 26 and articulation member 206 extending therethrough from the proximal end to the distal end. The drive member can extend generally centrally through the shaft assembly while the articulation member is laterally offset. The proximal end of the tubular shaft can include a coupling collar 410 for coupling to the coupler 46 at the distal end of the handle. In the illustrated embodiment, the shaft assembly can include a proximal shaft 'lock out' mechanism. The lockout mechanism comprises a locking ring positioned within a shaft coupler at the proximal end of the elongate shaft and at least one lockout member radially outwardly advanceable through the coupling collar 410. The lockout member can be biased radially outwardly, but held in a radially inward position by the locking ring in an initial position. When the proximal end of the shaft is coupled to a handle assembly in a rotation sequence corresponding to a bayonet connection, the locking ring is engaged with a mating surface in the handle assembly and rotates relative to the elongate shaft. This rotation of the locking ring releases the lockout member. Upon removal of the shaft from the handle assembly, the lockout member radially expands. In this expanded position, the lockout member interferes with recoupling the elongate shaft to the handle assembly. Thus, this lockout mechanism can serve to limit inadvertent reuse of an elongate shaft assembly.

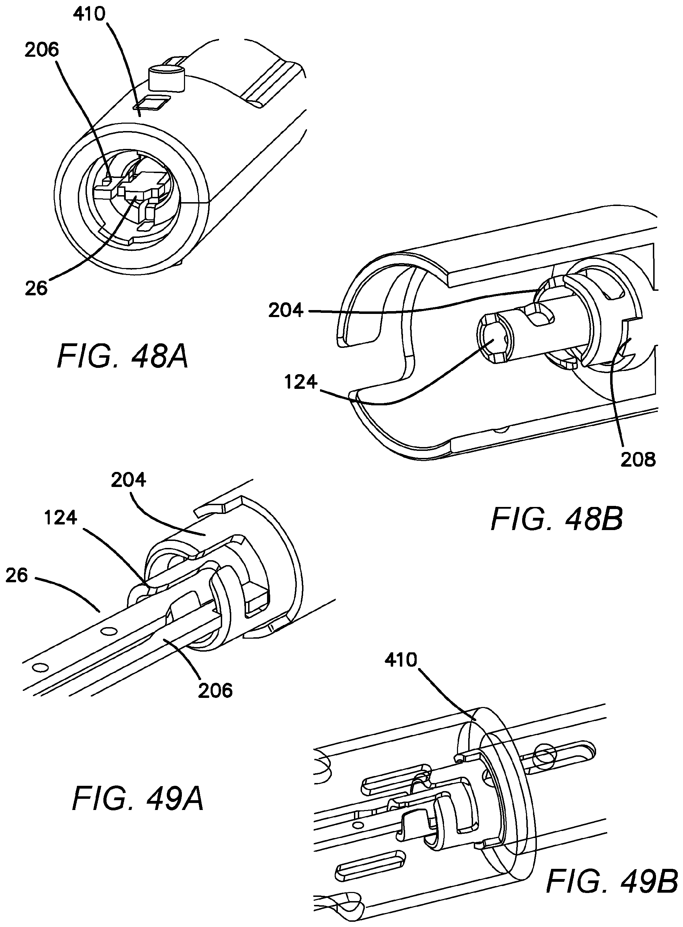

With reference to Figures 48A and 48B, engagement of the bayonet coupling between the shaft assembly and the handle is illustrated. The coupler of the handle can comprise a rotation sleeve for coupling to the coupling collar 410 in which an actuation adapter 124, an articulation adapter 204, and an identification sleeve 208 are positioned. During a bayonet coupling, the drive member of the shaft engages 26 with the actuation adapter 124, the articulation member 206 of the shaft engages with the articulation adapter 204, and a shaft identifier engages with the identification sleeve 208. Figures 49A and 49B illustrate the respective engagements with the shaft in a coupled configuration.

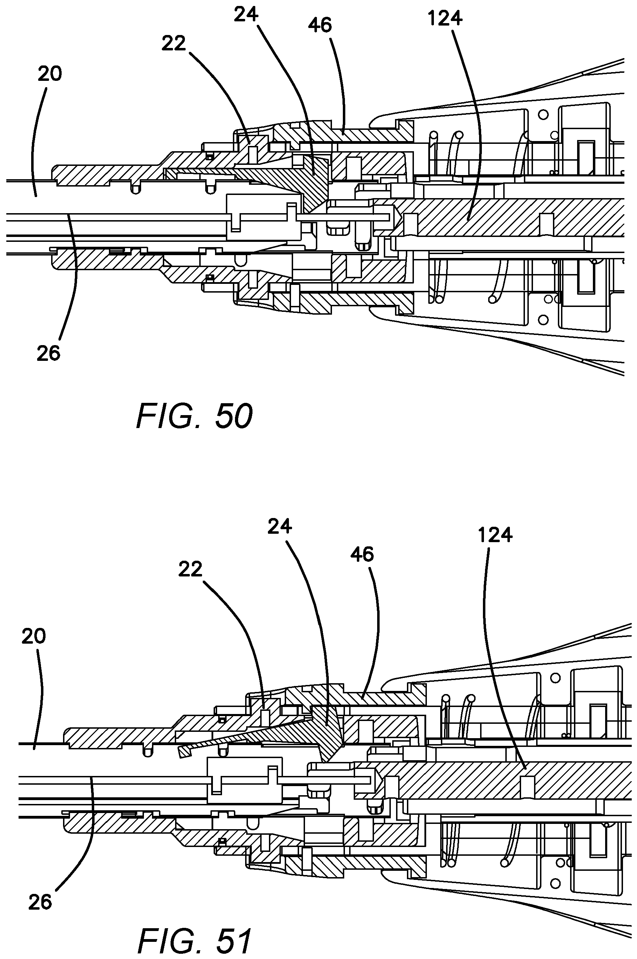

With reference to Figures 50 and 51, instead of or in addition to the lockout mechanism described with reference to Figures 46 and 47, certain embodiments of elongate shaft can include a lock-in or retention mechanism that operates upon initial distal advancement of the actuation adapter 124. As illustrated, a locking member 24 is pivotably coupled to a proximal end of the shaft 20. The locking member 24 can include a ramped or tapered lock surface at a proximal edge thereof. As illustrated in Figure 50, the shaft 20 is in a coupled, but unlocked configuration with respect to the coupler 46. In the coupled, unlocked configuration, the shaft 20 can be removed from the coupler 46 through the bayonet connection by a reverse of the sequence of operations of Figures 45A-45D. Once the actuation adapter 124 is advancing to operate the stapler, the actuation adapter 124 interacts with the ramped surface of the locking member 24 to advance the locking member radially outward into a locked position. In the locked position (Figure 51), the locking member 24 engages a locking ledge on the coupler 46 to lock in the shaft. With the shaft 20 locked in with respect to the handle 40, the shaft 20 cannot be removed from the handle 40 until the actuation adapter 124 has been returned to a fully proximally retracted position (typically corresponding to a return to a jaws open configuration following a full closure and stapling cycle of the jaw assembly).

Thus, the "lock In" feature prevents a user from removing the shaft from the handle once the drive member 26 has been driven forward. Once the locking member 24 is situated in the slot or ledge of a rotation insert of the coupler 46, a release knob of the coupler 46 is restricted from being pulled back. This locking action on the coupler prevents the user from rotating the shaft 20 out of the bayonet connection of the coupler 46.

With reference to Figure 52, a proximal end of the shaft assembly comprises a shaft coupler or coupling collar 410 positioned on the proximal end of the tubular shaft. Thus, the stapling system described herein can easily be adapted for use with shaft assemblies having various diameters. In some embodiments, an inner diameter the shaft coupler can be readily resized to accommodate various tubular shafts without requiring different handle assemblies to accommodate shaft assemblies of various diameters.

Although this application discloses certain preferred embodiments and examples, it will be understood by those skilled in the art that the present inventions extend beyond the specifically disclosed embodiments to other alternative embodiments and/or uses of the invention and obvious modifications and equivalents thereof. Further, the various features of these inventions can be used alone, or in combination with other features of these inventions other than as expressly described above. Thus, it is intended that the scope of the present inventions herein disclosed should not be limited by the particular disclosed embodiments described above, but should be determined only by a fair reading of the claims.

Other potentially novel aspects are defined by the following clauses.
Clause 1. A reload assembly for a surgical stapling system, the reload assembly comprising:
   an elongate shaft having a proximal end and a distal end and defining a longitudinal axis extending between the proximal end and the distal end;
   a jaw assembly positioned at the distal end of the elongate shaft, the jaw assembly comprising:
      a first jaw comprising a reload support configured to receive a staple reload; and
      a second jaw pivotably coupled to the first jaw, the second jaw comprising an anvil surface;
   a firing member longitudinally slidable within the jaw assembly;
   an actuation beam longitudinally slidable within the elongate shaft, the actuation beam having a proximal end and a distal end, the distal end of the actuation beam coupled to the firing member; and
   a reload lockout mechanism comprising:
      a lockout lever pivotally coupled to the reload support and pivotable between a locked position preventing distal movement of the actuation beam relative to the elongate shaft and an unlocked position allowing distal movement of the actuation beam relative to the elongate shaft.
Clause 2. The reload assembly of clause 1, wherein the lockout lever has a proximal end and a distal end and comprises a pivot between the proximal end and the distal end, the pivot defining a pivot axis of the lockout lever.
Clause 3. The reload assembly of clause 2, wherein the pivot axis is perpendicular to the longitudinal axis of the elongate shaft.
Clause 4. The reload assembly of clause 1, wherein the actuation beam comprises a lock recess formed therein.
Clause 5. The reload assembly of clause 4, wherein the lockout lever engages the lock recess when the lockout lever is in the locked position.
Clause 6. The reload assembly of clause 1, wherein the firing member has a tail portion extending proximally.
Clause 7. The reload assembly of clause 6, wherein the tail portion of the firing member engages the lockout lever to maintain the lockout lever in the unlocked position with the firing member in a proximally retracted position.
Clause 8. The reload assembly of clause 8, further comprising a staple reload positionable in the reload support, the reload comprising:
   a plurality of staples positioned therein, and
   a slider longitudinally advanceable within the reload from a proximal position to a distal position to deploy the staples from the reload, the slider comprising a slider tail extending proximally therefrom;
   wherein the slider tail engages the lockout lever with the slider in the proximal position to maintain the lockout lever in the unlocked position.
Clause 9. A reload assembly for a surgical stapling system, the reload assembly comprising:
   an elongate shaft having a proximal end and a distal end and defining a longitudinal axis extending between the proximal end and the distal end;
   a jaw assembly positioned at the distal end of the elongate shaft, the jaw assembly comprising:
      a first jaw comprising a reload support configured to receive a staple reload; and
      a second jaw pivotably coupled to the first jaw, the second jaw comprising an anvil surface;
      an actuation beam longitudinally slidable within the elongate shaft, the actuation beam having a proximal end and a distal end, the distal end of the actuation beam coupled to the jaw assembly; and
      a shaft coupler at the proximal end of the elongate shaft, the shaft coupler comprising a locking member positioned therein, the locking member radially outwardly advanceable by distal actuation of the proximal end of the actuation beam.
Clause 10. The reload assembly of clause 9 wherein the shaft coupler has an unlocked configuration with the locking member in a radially inward position, the shaft removably positionable in a coupler of a handle assembly in the unlocked configuration.
Clause 11. The reload assembly of clause 9, wherein the shaft coupler has a locked configuration with the locking member radially outwardly advanced.
Clause 12. The reload assembly of clause 9, wherein the locking member is pviotably coupled to the elongate shaft and pivotable from an unlocked position to a locked position.
Clause 13. The reload assembly of clause 10, wherein the locking member comprises a lever having a proximal end, the locking member comprising a tapered lock surface at the proximal end that is radially outwardly advanceable by distal actuation of the actuation beam.
Clause 14. A reload assembly for a surgical stapling system, the reload assembly comprising:
   an elongate shaft having a proximal end and a distal end and defining a longitudinal axis extending between the proximal end and the distal end;
   a jaw assembly positioned at the distal end of the elongate shaft, the jaw assembly comprising:
      a first jaw comprising a reload support configured to receive a staple reload; and
      a second jaw pivotably coupled to the first jaw, the second jaw comprising an anvil surface;
      an actuation beam longitudinally slidable within the elongate shaft, the actuation beam having a proximal end and a distal end, the distal end of the actuation beam coupled to the jaw assembly; and
      a shaft coupler at the proximal end of the elongate shaft, the shaft coupler configured to removably couple to a handle assembly, the shaft coupler comprising a lockout mechanism positioned therein,
      the lockout mechanism comprising a locking ring rotatable about the longitudinal axis; and
      a lockout member radially outwardly advanceable by rotation of the locking ring.
Clause 15. The reload assembly of clause 14, wherein the lockout member is extendable through a port in the shaft coupler to a locked position radially outwardly of the shaft coupler.
Clause 16. The reload assembly of clause 15, wherein with the lockout member in the locked position, the lockout mechanism interferes with coupling the shaft coupler to a handle coupler.
Clause 17. A reload assembly for a surgical stapling system, the reload assembly comprising:
   an elongate shaft having a proximal end and a distal end and defining a longitudinal axis extending between the proximal end and the distal end;
   a jaw assembly articulably coupled to the elongate shaft at the distal end of the elongate shaft, the jaw assembly comprising:
      a first jaw comprising a reload support configured to receive a staple reload;
      a second jaw pivotably coupled to the first jaw, the second jaw comprising an anvil surface;
      an actuation beam longitudinally slidable within the elongate shaft to actuate the jaw assembly, the actuation beam having a proximal end and a distal end;
      an articulation link longitudinally slidable within the elongate shaft to articulate the jaw assembly relative to the elongate shaft, the articulation link having a proximal end positioned adjacent the proximal end of the elongate shaft and a distal end pivotably coupled to the jaw assembly;
      a support link longitudinally slidable within the elongate shaft, the support link having a proximal end extending longitudinally to a distal end pivotably coupled to the jaw assembly; and
      an articulation latching mechanism positioned within the elongate shaft between the proximal end and the distal end, the articulation latching mechanism having an unlatched configuration in which the articulation link and the support link are slidable within the elongate shaft and a latched configuration wherein the articulation latching mechanism engages the articulation link and the support link to prevent longitudinal sliding of the articulation link and the support link.
Clause 18. The reload assembly of clause 17, wherein the articulation link comprises a first plurality of teeth formed thereon between the proximal end and the distal end of the articulation link, the support link comprises a second plurality of teeth formed thereon between the proximal end and the distal end of the support link and the articulation latching mechanism comprises:
   a first shoe having a first pawl surface formed thereon, the first shoe engageable with the first plurality of teeth;
   a second shoe having a second pawl surface formed thereon, the second shoe engageable with the second plurality of teeth; and
   a biasing member coupled to the first shoe and the second shoe and biasing the first shoe and the second shoe out of engagement with the first plurality of teeth and the second plurality of teeth.
Clause 19. The reload assembly of clause 18, wherein the actuation beam comprises a recess segment formed between the proximal end and the distal end of the actuation beam, a ramped segment proximal the recess segment, and a latching segment proximal the ramped segment.
Clause 20. The reload assembly of clause 19, wherein with the actuation beam in a proximal position, the first shoe and the second shoe are disposed radially outwardly of the recess segment and the articulation latching mechanism is in the unlatched configuration.
Clause 21. The reload assembly of clause 20, wherein upon distal movement of the actuation beam, the ramped segment and latching segment engage the first shoe and the second shoe are advanced radially outwardly into engagement with the first and second plurality of teeth to position the articulation latching mechanism in the latched configuration.
Clause 22. The reload assembly of clause 18, wherein the biasing member comprises a spring clip biasing the first shoe and the second shoe radially inwardly.
Clause 23. The reload assembly of clause 18, wherein the first pawl surface comprises a first plurality of teeth formed in the first shoe and extending radially outwardly from the first shoe.
Clause 24. The reload assembly of clause 23, wherein the second pawl surface comprises a second plurality of teeth formed in the second shoe and extending radially outwardly from the second shoe.
Clause 25. The reload assembly of clause 18, wherein the proximal end of the support link is disposed between the proximal end and the distal end of the elongate shaft.

## Claims

1. A reload assembly for a surgical stapling system, the reload assembly comprising:
an elongate shaft (20) having a proximal end and a distal end and defining a longitudinal axis extending between the proximal end and the distal end;
a jaw assembly (30) positioned at the distal end of the elongate shaft (20), the jaw assembly (30) comprising:
a first jaw (32) comprising a reload support (210) configured to receive a staple reload (50); and
a second jaw (34) pivotably coupled to the first jaw, the second jaw (34) comprising an anvil (220) surface;
an actuation beam (26) longitudinally slidable within the elongate shaft (20), the actuation beam (26) having a proximal end and a distal end, the distal end of the actuation beam coupled to the jaw assembly (30); and
a shaft coupler at the proximal end of the elongate shaft (20), the shaft coupler configured to removably couple to a handle assembly, the shaft coupler comprising a lockout mechanism positioned therein,
the lockout mechanism comprising a locking ring rotatable about the longitudinal axis; and
a lockout member radially outwardly advanceable by rotation of the locking ring.

2. The reload assembly of claim 1, wherein the lockout member is extendable through a port in the shaft coupler to a locked position radially outwardly of the shaft coupler.

3. The reload assembly of claim 2, wherein with the lockout member in the locked position, the lockout mechanism interferes with coupling the shaft coupler to a handle coupler of a handle assembly.

4. The reload assembly of any of claims 1-3, wherein the lockout member is biased radially outwardly and in an initial position, the lockout member is held in a radially inward position by the locking ring.

5. The reload assembly of any of the preceding claims, wherein the shaft coupler comprises a coupling collar (410) positioned at the proximal end of the elongate shaft (20).

6. The reload assembly of any of the preceding claims, wherein the proximal end of the elongate shaft (20) is coupled to the handle assembly in a rotation sequence corresponding to a bayonet connection in which the locking ring is engaged with a mating surface in the handle assembly and rotated relative to the elongate shaft.

7. The reload assembly of claim 6, wherein upon removal of the elongate shaft (20) from the handle assembly, the lockout member radially expands.

8. The reload assembly of any of the preceding claims, wherein the jaw assembly (30) further comprises a reload lockout mechanism (580).

9. The reload assembly of any of the preceding claims, wherein the jaw assembly (30) is coupled to the elongate shaft (20) at an articulation joint (300, 300').

10. The reload assembly of claim 9, wherein the articulation joint (300') further comprises an articulation latch mechanism (340) positioned within the elongate shaft (20).
